# EUROPEAN PATENT SPECIFICATION

(11) **EP 1 227 734 B1**
(45) Date of publication and mention of the grant of the patent: **12.01.2005**
(21) Application number: 00974202.4
(22) Date of filing: 03.11.2000
(51) Int. Cl.: A23D 9/007, A23D 9/02, A23L 1/30, A23L 2/52, A61K 31/575, A61P 3/06

(54) **COMPOSITIONS COMPRISING EDIBLE OILS OR FATS AND PHYTOSTEROLS AND/OR PHYTOSTANOLS DISSOLVED THEREIN**
ZUSAMMENSETZUNGEN MIT ESSBAREN ÖLEN ODER FETTEN UND DARIN AUFGELÖSTEN PHYTOSTEROLEN UND/ODER PHYTOSTANOLEN
COMPOSITIONS CONTENANT DES HUILES OU DES GRAISSES COMESTIBLES AINSI QUE DES PHYTOST ROLS ET/OU DES PHYTOSTANOLS DISSOUS

(30) Priority: 03.11.1999 US 434356
(43) Date of publication of application: 07.08.2002
(73) Proprietor: Forbes Medi-Tech Inc., Vancouver, British Columbia V6C 2T8 (CA)
(72) Inventor: ZAWISTOWSKI, Jerzy, Port Moody, British Columbia V3H 4K6 (CA)
(74) Representative: Jones, Helen Marjorie Meredith
(86) International application number: PCT/CA2000/001298
(87) International publication number: WO 2001/032029

(56) References cited:
- EP-A- 0 897 671
- EP-A- 1 005 859
- WO-A-97/42830
- WO-A-99/43218
- WO-A-99/56729
- WO-A-99/63841
- US-A- 4 218 334
- PATENT ABSTRACTS OF JAPAN vol. 1995, no. 02, 31 March 1995 (1995-03-31) -& JP 06 329588 A (RIKEN VITAMIN CO LTD), 29 November 1994 (1994-11-29)

## Description

### FIELD OF THE INVENTION

This present invention relates to the field of phytosterols and phytostanols and their incorporation into oils and fats.

### BACKGROUND OF THE INVENTION

While recent advances in science and technology are helping to improve quality and add years to human life, the prevention of atherosclerosis, the underlying cause of cardiovascular disease ("CVD") has not been sufficiently addressed. Atherosclerosis is a degenerative process resulting from an interplay of inherited (genetic) factors and environmental factors such as diet and lifestyle. Research to date suggest that cholesterol may play a role in atherosclerosis by forming atherosclerotic plaques in blood vessels, ultimately cutting off blood supply to the heart muscle or alternatively to the brain or limbs, depending on the location of the plaque in the arterial tree (1,2). Overviews have indicated that a 1 % reduction in a person's total serum cholesterol yields a 2% reduction in risk of a coronary artery event (3). Statistically, a 10% decrease in average serum cholesterol (e.g. from 6.0 mmol/L to 5.3 mmol/L) may result in the prevention of 100,000 deaths in the United States annually (4).

Sterols are naturally occurring compounds that perform many critical cellular functions. Phytosterols such as campesterol, stigmasterol and beta-sitosterol in plants, ergosterol in fungi and cholesterol in animals are each primary components of cellular and sub-cellular membranes in their respective cell types. The dietary source of phytosterols in humans comes from plant materials i.e. vegetables and plant oils. The estimated daily phytosterol content in the conventional western-type diet is approximately 60-80 milligrams in contrast to a vegetarian diet which would provide about 500 milligrams per day.

Phytosterols have received a great deal of attention due to their ability to decrease serum cholesterol levels when fed to a number of mammalian species, including humans. While the precise mechanism of action remains largely unknown, the relationship between cholesterol and phytosterols is apparently due in part to the similarities between the respective chemical structures (the differences occurring in the side chains of the molecules). It is assumed that phytosterols displace cholesterol from the micellar phase and thereby reduce its absorption or possibly compete with receptor and/or carrier sites in the cholesterol absorption process.

Over forty years ago, Eli Lilly marketed a sterol preparation from tall oil and later from soybean oil called Cytellin™ which was found to lower serum cholesterol by about 9% according to one report (5). Various subsequent researchers have explored the effects of sitosterol preparations on plasma lipid and lipoprotein concentrations (6) and the effects of sitosterol and campesterol from soybean and tall oil sources on serum cholesterols (7). A composition of phytosterols which has been found to be highly effective in lowering serum cholesterol is disclosed in US Patent Serial No. 5,770,749 to Kutney et al. and comprises no more than 70% by weight beta-sitosterol, at least 10% by weight campesterol and stigmastanol (beta-sitostanol). It is noted in this patent that there is some form of synergy between the constituent phytosterols, affording even better cholesterol-lowering results than had been previously achieved.

Despite the obvious and now well recorded advantages of phytosterols, not only in the treatment of CVD and its underlying conditions such as hypercholesterolemia, hyperlipidemia, atherosclerosis, hypertension, thrombosis but in the treatment of other diseases such as Type II diabetes, dementia cancer and aging , the administration of phytosterols and the incorporation thereof into foods, beverages pharmaceuticals and other delivery vehicles has been complicated by the fact that they are highly hydrophobic, water insoluble and they are also very difficult to dissolve homogenously in oils and fats. Studies have investigated how the form (for example crystalline, suspension, granular) in which the phytosterols are dosed impacts on their ability to lower serum cholesterol levels. As they are highly hydrophobic, phytosterol crystals exhibit poor solubility in the micellar phase in the digestive tract and therefore are not capable of efficiently blocking cholesterol absorption. Since solubilization of phytosterols may significantly improve the inhibition of cholesterol absorption, adaptations must be made in this respect.

Early research focused on grinding or milling the phytosterols in order to enhance their solubility (US Patent Serial Nos: 3,881,005 and 4,195,084 both to Eli Lilly). In addition, researchers have looked to the esterification of phytosterols in order to enhance their solubility. German Patent 2035069/January 28, 1971 (analogous to US Patent No. 3,751,569) describes the addition of phytosterol fatty acid esters to cooking oil. The esterification is carried out between a free sterol and a fatty acid anhydride, with perchloric acid as the catalyst. The significant drawback to this process, along with others, is the use of non-food grade catalysts and reagents.

US Patent Serial No. 4,588,717 to the David E. Mitchell Medical Research Institute describes a vitamin supplement which comprises a fatty acid ester of a phytosterol, wherein the fatty acid ester has from about 18 to 20 carbon atoms in the main carbon chain.

US Patent Serial No. 5,502,045 to Raision Tehtaat Oy AB describes the preparation of a beta-sitostanol fatty acid ester mixture. Although the attempt of this patent is to produce a soluble and stable phytostanol delivery system, there are some problems with the long term stability of these "fatty acid" esterified products due to the ultimate oxidation of the unsaturated fatty acid moiety.

US Patent Serial No. 3,865,939 to The Proctor & Gamble Company teaches edible oil compositions comprising plant sterols, in which the sterols are dissolved by way of fatty acids having length in the range of six to 18 carbon atoms. This patent focuses on plant sterols as opposed to stanols the latter of which, as noted above, are inherently more difficult to solubilize in oils.

Accordingly, the provision of a method to dissolve phytosterols/phytostanols in oils and fats resulting in a product which could be used per se or incorporated without further modification into delivery vehicles would be highly desirable and has not heretofore been satisfactorily achieved.

It is an object of the present invention to obviate or mitigate the above disadvantages.

### SUMMARY OF THE INVENTION

The present invention provides a method of producing an edible oil or fat composition comprising one or more phytosterols and/or phytostanols, and in which these phytosterols or phytostanols are substantially completely dissolved, which comprises:
a) heating the phytosterols and/or phytostanols to form a molten material;
b) heating the edible oil or fat;
c) mixing the molten material with the heated edible oil or fat; and
d) cooling the composition so formed.

The present invention also comprises a method of dissolving phytosterols and/or phytostanols in edible oils or fats which comprises following steps a) to d) as set out above.

The present invention further comprises an edible oil or fat composition comprising substantially completely dissolved phytosterols and/or phytostanols prepared by:
a) heating the phytosterols and/or phytostanols to form a molten material;
b) heating the edible oil or fat;
c) mixing the molten material with the heated edible oil or fat; and
d) cooling the composition so formed.

The present invention further provides foods, beverages, dietary supplements and nutraceuticals comprising an edible oil or fat composition having phytosterols and/or phytostanols substantially completely dissolved therein.

The present invention further provides a method for treating or preventing CVD and its underlying conditions including atherosclerosis, hypercholesterolemia, hyperlipidemia, hypertension, thrombosis, and related diseases such as Type II diabetes, as well as other diseases that include oxidative damage as part of the underlying disease process such as dementia, aging, and cancer by administering to an animal, such as a human, an oil or fat composition having phytosterols and/or phytostanols substantially completely dissolved therein or by administering to the animal a derivative product such as a food, beverage or nutraceutical comprising an oil or fat composition having phytosterols and/or phytostanols substantially completely dissolved therein.

What is achieved within the scope of the present invention is greatly enhanced solubility of phytosterols and/or stanols in edible oils and fats by a method which is simple, economical and which does not require extensive prior modifications to the sterols or stanols, such as by esterification. Naturally occurring and isolated phytosterols and stanols are in coarse, powder, crystalline form which is not amenable to the formation of a homogeneous mixture in water, oils or fats, without some type of modification. Although esterification of phytosterols and stanols does make them considerably more soluble in fats and oils and is a widely used technique for practical reasons, these esterified derivatives do not inhibit the absorption of cholesterol as effectively as free sterols (7b). In a number of prior patents, stanols, in particular, are esterified in order to enhance their poor solubility (see US Patent Serial No. 5,502,045 to Raision Tehtaat Oy AB). Heating beta-sitosterol in oil is described in PCT/FI99/00121; however, the resultant product is one in which the sterols are only partially dissolved.

Furthermore, within the scope of the present invention, no solvents, some of which have questionable human safety, are required to enhance dissolution of the phytosterols/stanols. The key to the success of the homogeneous dissolution, as achieved herein, is the preparation of molten or melted phytosterols that are mixed with oils or fats under appropriate conditions. This enhanced solubility in oils and fats allows the use of these media per se without any further enhancements or modifications. Accordingly, the oil and fat compositions of the present invention can be prepared and used as such or they can be easily incorporated into foods, beverages, dietary supplements and nutraceuticals. This enhanced solubility generally translates into lower concentrations of phytosterols and/or phytostanols that need be provided in the oil or fat in order to achieve the desired therapeutic or dietary effect.

### PREFERRED EMBODIMENTS OF THE INVENTION

Although the dietary and therapeutic benefits of phytosterols and phytostanols are widely recognised, a problem which has continually beset the art is the inherently non-absorbable nature of plant steroids in edible oils and fats. According to one aspect of the present invention, there is provided a method of producing a composition comprising an edible oil or a fat and one or more phytosterols and/or phytostanols, and in which these phytosterols or phytostanols are substantially completely dissolved, which comprises:
a) heating the phytosterols and/or phytostanols to form a molten material;
b) heating the edible oil or fat;
c) mixing the molten material with the heated edible oil or fat; and
d) cooling the composition so formed.

Each of the components of the composition, namely: the phytosterol or stanol and the edible fat or oil is described in more detail below. Also described are preferred or recommended procedures for achieving the desired level of steroid solubility and preferred or recommended procedures for incorporating the edible oil/fat compositions into food, pharmaceutical or nutraceutical "delivery" vehicles. Lastly, there is presented a series of non-limiting examples featuring the preparation of some compositions of the present invention.

What is achieved within the scope of the present invention, in one aspect, is the solubilization of phytosterols and more importantly phytostanols, in oils without compromising the clarity of the oils i.e. the oils so formed are clear at room temperature. In another aspect of the present invention, phytosterols and phytostanols are solubilized in animal fats that become solid at room temperature i.e. when cooled. This method allows uniform distribution of the phytosterols/stanols in the solid fat, which has not heretofore been adequately achieved, and concomitantly enhances the bioavailablity of the phytosterols/stanols.

### Phytosterols/Phytostanols

As used herein, the term "phytosterol" includes all phytosterols without limitation, for example: sitosterol, campesterol, stigmasterol, brassicasterol, desmosterol, chalinosterol, poriferasterol, clionasterol and all natural or synthesized forms and derivatives thereof, including isomers. The term "phytostanol" includes all saturated or hydrogenated phytosterols and all natural or synthesized forms and derivatives thereof, including isomers. It is to be understood that modifications to the phytosterols and phytostanols i.e. to include modified side chains also falls within the purview of this invention. It is also to be understood that, when in doubt throughout the specification, the term "phytosterol" encompasses both phytosterol and phytostanol i.e. the terms may be used interchangeably unless otherwise specified.

The phytosterols and phytostanols for use in forming derivatives in accordance with this invention may be procured from a variety of natural sources. For example, they may be obtained from the processing of plant oils (including aquatic plants) such as corn oil and other vegetable oils, wheat germ oil, soy extract, rice extract, rice bran, rapeseed oil, sunflower oil, sesame oil and fish (and other marine-source) oils. The present invention is not to be limited to any one source of phytosterols or phytostanols. US Patent Serial No. 4,420,427 teaches the preparation of sterols from vegetable oil sludge using solvents such as methanol. Alternatively, phytosterols and phytostanols may be obtained from tall oil pitch or soap, by-products of forestry practises as described in US Patent Serial No.5,770,749.

In one preferred form, the phytosterols/stanols which are dissolved into the oil or fat are naturally-derived or synthesized beta-sitosterol, campestanol, sitostanol and campesterol. In another preferred form, the phytosterols/stanols which are dissolved into the oil or fat are naturally-derived or synthesized sitostanol or naturally derived or synthesized campestanol or mixtures thereof.

### Oils/Fats

A wide variety of edible oils and fats can be used in dissolving the phytosterols in accordance with the present invention. This includes any food-grade or nutraceutical-grade oily or fatty substance, of plant or animal or marine origin, or mixture thereof. Without limiting the generality of the foregoing, all salad and cooking oils, including sunflower oil, rapeseed oil, soybean oil, olive oil, corn oil, safflower oil, sesame seed oil may be used. Oils obtained by directed low temperature interesterification or rearrangement of animal or vegetable fatty materials, followed by removal of higher melting solids may also be used. The fats include all animal fats.

### Methods of Preparation

The key feature of the method-invention as described herein is the heating of the phytosterols to form a molten material prior to mixing with the heated oil or fat. Generally, phytosterols/stanols may be heated to this molten condition at a temperature of from about 120° to 160° C, most preferably from about 135° to 145° C. Although the present invention is not so limited, the melting point of most phytosterols is about 138-140° C. In one embodiment, no additional material need be added to the molten phytosterol material. This molten material so formed is then added to oil or fat which has been previously heated to a temperature of from about 90° to 150° C, more preferably from about 100° to 120° C. The oil/fat "composition" comprising the molten phytosterols is then cooled to room temperature. The resultant product is an oil or fat in which the phytosterols are and remain substantially completely dissolved at room temperature. The colouring of the "oil" product, which is liquid, is yellow (pale or light) and transparent with no visually discernible crystal precipitate. The "fat" product is solid at room temperature.

In a preferred form, the composition of the present invention comprises from 1% to 30% phytosterol and from 99% to 70% oil or fat (hereinafter, the term % or percentage will refer to % or percentage by weight unless otherwise specified). More preferably, the composition comprises from 2% to 10% phytosterol and from 98% to 90% oil or fat. Generally, it is expected with respect to edible cooking oils, adapted as described herein in order to comprise phytosterols, that the amount of phytosterols will be 5% or less and the amount of oil 95% or greater. Alternatively, in using oils or fats to prepare delivery vehicles (foods, beverages, pharmaceutical and the like as described further below), it may be desirable to have a higher concentration of phytosterols dissolved therein. For example, the oil compositions used to prepare emulsions for spreads/margarines may have over 5% phytosterols. In this particular embodiment, it is preferred that one or more emulsifying agents be included in the composition as described below.

### Emulisifying Agents

Optionally, one or more emulsifiers may be mixed with the phytosterols and/or phytostanols prior to the melting step. These emulsifiers include, but are not limited to (wherein bracketed numerals refer to the preferred HLB values): anionic surfactants such as alcohol ether sulfates, alkyl sulfates (30-40), soaps (12-20) and sulfosuccinates; cationic surfactants such as quaternary ammonium compounds; zwitterionic surfactants such as alkyl betaine derivatives; amphoteric surfactants such as fatty amine sulfates, difatty alkyl triethanolamine derivatives (16-17); and nonionic surfactants such as the polyglycol ether derivatives of aliphatic or cycloaliphatic alcohols, saturated fatty acids and alkyphenols, water-soluble polyethyleneoxy adducts onto polypropylene glycol and alkyl polypropylene glycol, nonylphenol polyethoxyethanols, castor oil polyglycol ethers, polypropylene/polyethylene oxide adducts, tributylphenoxy-polyethoxyethanol, polyethylene glycol, octylphenoxy-polyethoxyethanol, lanolin alcohols, polyoxyethylated (POE) alkyl phenols, POE fatty amides, POE fatty alcohol ethers, POE fatty amines, POE fatty esters, poloxamers (7-19), POE glycol monoethers (13-16), polysorbates and sorbitan esters. More specifically, the emulsifiers include: glycerin fatty acid esters, diglycerin fatty acid esters, polyglycerin fatty acid esters, organic acid glycerin fatty acid esters, propylene glycol fatty acid esters, sorbitan fatty acid esters and sucrose fatty acid esters. This list is not intended to be exhaustive as other emulsifiers are equally suitable. Most preferred as emulsifiers are lecithin and phospholipids. The addition of an emulsifier is most recommended wherein the concentration of phytosterols in the oil or fat composition is to exceed 5%, otherwise the addition should generally be unnecessary. It is preferred that the amount of emulsifier included be in the range of 0.01 to 10% w/w, more preferably in the range of 0.2 to 5% w/w.

### Methods of Use

The oil and fat compositions of the present invention, comprising substantially completely dissolved phytosterols, may be used directly and without further modification in cooking, baking and the like as an agent to lower serum cholesterol in animals, particularly humans. Alternatively, the composition may be treated to enhance delivery into various other delivery media. For example, the present invention fully contemplates the formation of oleaginous gel foodstuffs such as peanut butter, mayonnaise, ice cream and margarine spreads incorporating such compositions. There are numerous modes or "vehicles" of delivery of this composition, accordingly, this invention is not intended to be limited to the following delivery examples.

### 1) Pharmaceutical Dosage Forms:

It is contemplated within the scope of the present invention that the composition of the present invention may be incorporated into various conventional pharmaceutical preparations and dosage forms such as tablets (plain and coated) for use orally, bucally or lingually, capsules (hard and soft, gelatin, with or without additional coatings) powders, granules (including effervescent granules), pellets, microparticulates, solutions (such as micellar, syrups, elixirs and drops), lozenges, pastilles, ampuls, emulsions, microemulsions, ointments, creams, suppositories, gels, and transdermal patches, modified release dosage forms together with customary excipients and/or diluents and stabilizers.

The composition of the present invention, adapted into the appropriate dosage form as described above may be administered to animals, including humans, orally, by injection (intra-venously, subcutaneously, intra-peritoneally, intra-dermally or intra-muscularly), topically or in other ways. Although the precise mechanism of action is unclear, the composition of the present invention, administered intra-venously, lowers serum cholesterol. It is believed that some blends of phytosterols, in concert, may have, in addition to the role as an inhibitor of cholesterol absorption in the intestine, a systemic effect on cholesterol homeostasis through bile acid synthesis, enterocycte and biliary cholesterol excretion, bile acid excretion and changes in enzyme kinetics and cholesterol transport between various compartments within the body (PCT/CA97/00474 which was published on January 15, 1998).

The oil and fat compositions as described herein may be used in both dietary and therapeutic capacities in order to treat and/or prevent CVD, its underlying conditions such as hypercholesterolemia, hyperlipidemia, arteriosclerosis, hypertension, thrombosis, related diseases such as Type II diabetes, as well as other diseases that include oxidative damage as part of the underlying disease process such as dementia, aging, and cancer. In populations, which are considered "high-risk" for CVD or any of the oxidation related disorders, it is contemplated that the compositions and foodstuffs in which they are contained be used in primary, secondary and tertiary treatment programs.

In order to appreciate the various possible vehicles of the delivery of the compositions, the list below is provided. The doses of the compositions will vary depending upon, among other factors, the mode of delivery (i.e. how and into which food or beverage or pharmaceutical the composition is ultimately incorporated) , the patient size and condition, the result to be achieved, as well as other factors known to those skilled in the art of food additives and medicinal agents. Generally, however, it is preferred that the compositions of the present invention be administered to humans in a form comprising up to 6 grams (based on a 70kg person) of phytosterols and/or phytostanols per day, more preferably from 1-5 grams per day and most preferably 1.5 grams per day. It will also be recognized that the provision of much larger daily doses of the derivatives are not harmful to the animal host, as excess will simply pass through normal excretory channels.

### Foods/Beverages/Nutraceuticals :

One primary purpose of the present invention is to create modified edible oil and fat compositions which can be used *per se* in cooking, frying and the like without further modification. Alternatively, the compositions of the present invention may be incorporated into or otherwise used in the preparation of foods, beverages and nutraceuticals, including, without limitation, the following:
1) Fat-Based Products--such as margarines, spreads, peanut butter, peanut spreads, mayonnaise (many of which are formed using emulsions), shortenings, cooking and frying oils and dressings;
2) Grain-based Goods-for example, bread and pastas, cookies, pastries, whether these goods are cooked, baked or otherwise processed;
3) Confectioneries--such as chocolate, candies, chewing gum, desserts, non-dairy toppings (for example Cool Whip™), sorbets, dairy and non-dairy shakes, icings and other fillings;
4) Beverages- dietary supplement and meal replacement drinks such as those sold under the trade-marks Boost™ and Ensure™; and any drinkable emulsions which contain added fat or oils;
5) Miscellaneous Products--including processed foods such as soups, pre-prepared pasta sauces, pre-formed meals and the like; and
6) Dairy Products--butter, dairy spreads, and beverages such as shakes and any emlusions containing added fat or oils.

### EXAMPLES

The present invention is described by the following non-limiting examples:

### Example 1 Dissolution in Oils--Phytosterols in Soybean Oil

A number of compositions were prepared comprising phytosterols dissolved in oil, without the necessity of emulsifiers.
A) A mixture of phytosterols (hereinafter called "3P6") which comprises beta-sitosterol, campesterol, campestanol and sitostanol (the latter at about 33-40% w/w) was selected for dissolution in soybean oil. 0.5 grams of 3P6 (5% w/w) was heated to a molten condition under the heat of an oil bath at approximately 140° C. The molten phytosterol blend was added to 9.5 grams soybean oil (95%w/w) at approximately 140° C. The final composition was mixed for about 2 minutes while the temperature was reduced to 100-110° C.
B) 0.6 grams of 3P6 (6% w/w) was heated to a molten condition under the heat of an oil bath at approximately 140° C. The molten phytosterol blend was added to 9.4 grams soybean oil (94%w/w) at approximately 140° C. The final composition was mixed for about 2 minutes while the temperature was reduced to 100-110° C.
C) 0.7 grams of 3P6 (7% w/w) was heated to a molten condition under the heat of an oil bath at approximately 140° C. The molten phytosterol blend was added to 9.3 grams soybean oil (93%w/w) at approximately 140° C. The final composition was mixed for about 2 minutes while the temperature was reduced to 100-110° C.
D) 0.8 grams of 3P6 (8% w/w) was heated to a molten condition under the heat of an oil bath at approximately 140° C. The molten phytosterol blend was added to 9.2 grams soybean oil (92%w/w) at approximately 140° C. The final composition was mixed for about 2 minutes while the temperature was reduced to 100-110° C.
E) 0.9 grams of 3P6 (9% w/w) was heated to a molten condition under the heat of an oil bath at approximately 140° C. The molten phytosterol blend was added to 9.1 grams soybean oil (91 %w/w) at approximately 140° C. The final composition was mixed for about 2 minutes while the temperature was reduced to 100-110° C
F) 1.0 grams of 3P6 (10% w/w) was heated to a molten condition under the heat of an oil bath at approximately 140° C. The molten phytosterol blend was added to 9.0 grams soybean oil (90%w/w) at approximately 140° C. The final composition was mixed for about 2 minutes while the temperature was reduced to 100-110° C.

The 5% w/w phytosterol composition so formed yielded a clear solution with no discernible precipitation or crystals and was the most preferred composition without emulsifiers. Control sample compositions were prepared having comparable % ratios of the components, but made simply by adding the oil and phytosterols together with heat applied for approximately 3 minutes. As compared to the control samples, each of the compositions prepared by the method of the present invention was more clear indicating greater dissolution of the phytosterols..

### Example 2: Dissolution in Oils --Phytosterols and Emulsifiers in Soybean Oil

Three types of emulsifiers were tested: EMULTOP ™(a lyso-PC enriched lecithin); EPIKURON 200™ (containing over 98% phospholipids) and Phosphoderm™ (approximately 80% phospholipids in alcohol). Each of these three were tested at amounts ranging from 0.01 % w/w to 1 % w/w. Phytosterol amounts ranged from 5-10% w/w. The protocol described below for Epikuron at each set amount and phytosterols at 5% w/w is applicable to all compositons, substituting, of course, the different percentages of the three components
A) A mixture of phytosterols ("3P6" as described in Example 1) was selected for dissolution in soybean oil. 0.5 grams of 3P6 (5% w/w) was heated to a molten condition under the heat of an oil bath at approximately 140° C. 0.1 grams of Epikurion 200 (1%w/w), an emulsifier with over 98% phospholipids was added and stirred into the melted phytosterols. After being well mixed, the phytosterol/phospholipids were added to 9.4 grams soybean oil (94%w/w) at approximately 140° C. The final composition was mixed for about 2 minutes while the temperature was reduced to 100-110° C.
B) 0.5 grams of 3P6 (5%w/w) was heated to a molten condition under the heat of an oil bath at approximately 140° C. 0.05 grams of Epikurion 200 (0.5%) was added and stirred into the melted phytosterols. After being well mixed, the phytosterol/phospholipids were added to 9.45 grams soybean oil (94.5%) at approximately 140° C. The final composition was mixed for about 2 minutes while the temperature was reduced to 100-110° C.
C) 0.5 grams of 3P6 (5%w/w) was heated to a molten condition under the heat of an oil bath at approximately 140° C. 0.03 grams of Epikurion 200 (0.3%) was added and stirred into the melted phytosterols. After being well mixed, the phytosterol/phospholipids were added to 9.47 grams soybean oil (94.7%) at approximately 140° C. The final composition was mixed for about 2 minutes while the temperature was reduced to 100-110° C.
D) 0.5 grams of 3P6 (5%w/w) was heated to a molten condition under the heat of an oil bath at approximately 140° C. 0.01 grams of Epikurion 200 (0.1%) was added and stirred into the melted phytosterols. After being well mixed, the phytosterol/phospholipids were added to 9.49 grams soybean oil (94.9%) at approximately 140° C. The final composition was mixed for about 2 minutes while the temperature was reduced to 100-110° C.
E) 0.5 grams of 3P6 (5%w/w) was heated to a molten condition under the heat of an oil bath at approximately 140° C. 0.001 grams of Epikurion 200 (0.01 %) was added and stirred into the melted phytosterols. After being well mixed, the phytosterol/phospholipids were added to 9.499 grams soybean oil (94.99%) at approximately 140° C. The final composition was mixed for about 2 minutes while the temperature was reduced to 100-110° C.
F) 0.5 grams of 3P6 (5%w/w) was heated to a molten condition under the heat of an oil bath at approximately 140° C. The molten phytosterol blend was added to 9.5 grams soybean oil (95%) at approximately 140° C. The final composition was mixed for about 2 minutes while the temperature was reduced to 100-110° C.

The best results i.e. clearest compositions with no discernible precipitation or crystals were obtained with 7% w/w phytosterol or less and 0.30% w/w lecithin (EMULTOP) as emulisifer and with 8% w/w or less phytosterol and 0.30% w/w phospholipids (PHOSPHODERM) in alcohol as emulsifier. As compared to control sample compositions (having comparable % ratios of phytosterols and oils without the emulsifiers); however, each of the compositions prepared by the method of the present invention and with emulsifier addition was more clear indicating greater dissolution of the phytosterols.

### Example : 3 Dairy beverage

A phytosterol blend which consists of campesterol, campestanol, β-sitosterol and sitostanol was dissolved in oil as per Example 1. Xantham gum (0.1%), skim powder milk (8-12%) were combined with skim milk and permitted to remain at room temperature for 30 minutes to rehydrate powder milk. Next, a blend slowly mixed using an overhead stirrer such as Caframo equipped with a pitched blade impeller until uniform dispersion was obtained. Phytosterols containing oil was heated to 80 °C and added to the mixture while steering. Resulted mixture was than homogenized using a high sheer batch mixer (Ultra-Turrax T50 equipped with the dispersing element S50N, IKA Works Inc., Wilmington, NC, USA)..). Other devices such as a single-stage homogeniser, a two-stage homogeniser or a high-pressure microfluidizer may alternatively be used for homogenization of the milk mix. Next, milk mix was submitted to UHT treatment (141 °C 4 sec) and packed in aseptic containers for use as a beverage or pasteurized (69°C, 30 min) for further processing.

This dairy beverage may be used as a "base" to prepare any number of food and beverage products. Although the preparation of yogurt is shown by way of example below, other products such as cheese may equally be prepared.

### Example: 4 Yogurt

Pasteurized Phytrol containing dairy beverage (Example 3) was used to produced yogurt. Milk was standardized to 0.75 - 1% fat, 12 - 13% solids and 0.5-1 % of the phytosterol blend using the Pearsons Square method (Hyde, K.A. and Rothwell, J., 1973, In Ice Cream, Churchill Livingstone Ltd., London, U.K). About 3% by weight of active yogurt culture containing Lactobacillus bulgaricus and *Streptococcus thermophilus* in the ratio 1:1 were carefully introduced into warm milk mix. After gentle mixing, the inoculated milk was distributed into 125 g-containers filling to near top. The containers were thermally sealed with aluminum leads and placed in incubator (44°C) equipped with good uniform air circulator and temperature controller. Filled containers were permitted to remain at 44°C for 3-5 hours, until a firm, smooth gel was formed. During incubation, pH was monitored periodically. When pH reached about 4.5, yogurt was withdrawn from the incubator, chilled quickly and stored at 4°C.

### Example: 5 Non-dairy beverage

A phytosterol blend which consists of campesterol, campestanol, β-sitosterol and sitostanol was dissolved in oil as per Example 1. Xanthan gum (0.1 - 0.2%), Tween 65 (0.5-0.7%) and flavours were combine with water and slowly mixed using an overhead stirrer such as Caframo equipped with a pitched blade impeller at room temperature until uniform dispersion was obtained. Phytosterols containing oil was heated to 80 °C and added to aqueous mixture while steering. The mixture was homogenised and heat treated as described in Example 3.

### Example: 6 Bread

Breads containing 0.6% and 1.2% of the phytosterol blend comprising campesterol, campestanol, β-sitosterol and sitostanol (hereinafter referred to as "Phytrol") dissolved in oil (Crisco™) as per Example 1 were prepared using bread maker (Black & Decker, Model # B2005). The phytosterol composition (Crisco plus phytosterols) was mixed with the other ingredients in proportions indicated below.

| **Ingredients** | 0.6% Phytrol (g) | 1.2% Phytrol (g) |
|---|---|---|
| **Milk** | 334.00 | 334.00 |
| **Salt** | 7.50 | 7.50 |
| **Sugar** | 7.10 | 7.10 |
| **Crisco** | 12.00 | 12.00 |
| **Flour** | 535.00 | 535.00 |
| **Phytrol** | 5.42 | 10.84 |
| **Yeast** | 2.80 | 2.80 |

Ingredients were combined in the baking pan of bread maker. Preparation of dough and baking was conducting according the manufacturing instructions.

### Example : 7 Cereal Bar

Cereal bars of total weight 20g, and 40g that contained 3%, and 1.5% of Phytrol, respectively, were prepared. Phytrol® consisted of campesterol, campestanol, β-sitosterol and sitostanol was dissolved in partially hydrogenated vegetable oil using the protocol of Example 1. The oil/Phytrol blend was cooled to 30°C and emulsified using a high sheer batch mixer (Ultra-Turrax T50 equipped with the dispersing element S50N, IKA Works Inc., Wilmington, NC, USA). Subsequently, two oil blends (9.4% and 18.8% of Phytrol) were further emulsified using a high-pressure microfluidizer at 20,000 PSI.

Cereal bars were produced by combining binder (40%), water (5%) and edible particles (55%). Below two typical examples of binder used for making a cereal bar.

### Sucrose containing binder

| | |
|---|---|
| Phytrol (9.4% or 18.8%) containing oil | 40% |
| Sucrose | 22% |
| Water | 28% |
| Sodium Caseinate | 5% |
| Lecithin | 2% |
| Glycerin | 3% |

### Glucose containing binder

| | |
|---|---|
| Phytrol (9.4% or 18.8%) containing oil | 40% |
| Glucose syrup | 50% |
| Sodium Caseinate | 5% |
| Lecithin | 2% |
| Glycerin | 3% |

Sucrose in water /glucose syrup was heated to 100°C while Phytrol containing fat was liquefied at 40-80°C. Hot sugar solution was placed in the bowl (Hobart mixer, Model N50) and fat was added followed by adding all remaining binder ingredients. All ingredients were thoroughly and vigorously mixed. After cooling down to 40°C, edible particles are added while thorough, non-vigorous mixing was carried out. Following edible particles were typically incorporated into the cereal bars.

### Edible particles

| | |
|---|---|
| Rolled oats | 20-40% |
| Crisped rice | 10-20% |
| Puffed barley | 10-20% |
| Dried apple dices | 10-20% |
| Shredded coconut | 5-10% |
| Raisins | 5-10% |
| Various nuts | 5-10% |

After mixing was completed, mixed material was placed in the forming mold and pressed with a roller. After removal from the mold, it was cut into ready to eat various sizes cereal bars.

### Example : 8 Spread

Light margarine (60% fat) containing 6% of Phytrol was produced in batches of 5-10kg. Phytrol® consisted of campesterol, campestanol, β-sitosterol and sitostanol was dissolved in the oils using the protocol outlined in Example 1. Clear fat solution was placed in the feeding tank (20L), cooled to 40-45 oC and stirred using (Ultra-Turrax T50 equipped with the dispersing element S50N, IKA Works Inc., Wilmington, NC, USA). Next, the water fraction (40%) was added and temperature was adjusted to 60 oC. The blend was submitted into a votator and processed at 8-10°C. The composition of margarine is described below.

| **Ingredient** | **Wt%** |
|---|---|
| **Water Phase** | |
| **Water** | 39.0 |
| **Salt** | 1.0 |
| **Potassium sorbate** | 0.001 |

| **Oil Phase** | |
|---|---|
| **Soybean oil** | 38.025 |
| **Palm kernel oil** | 15.0 |
| **Phytrol** | 6.0 |
| **Mono/diglycerides** | 0.6 |
| **Lecithin** | 0.15 |
| **Flavor** | 0.075 |
| **Beta-carotene** | 0.15 |

### Example : 9 Chocolate

Milk chocolate containing 6% of Phytrol was produced in batches of 20-50kg. Phytrol® consisted of campesterol, campestanol, β-sitosterol and sitostanol was dissolved in soybean oil using the protocol outlined in Example 1. The blend (20% Phytrol) was subsequently emulsified using a high-pressure microfluidizer at 20,000 PSI. Chocolate was composed of an outer shell (42 wt%, no Phytrol) and a center (69%, Phytrol). Chocolate outer shell was made by mixing sugar (45%), whole milk powder (20%), cocoa butter (23%), cocoa mass (12%), soy lecithin (0.3%) and pure vanilla (0.1 %) in a heating tank. All ingredients were melted, tempered and deposited into molds. Center was prepare my mixing sugar, cocoa butter, whole milk powder, cocoa mass, soy lecithin and pure vanilla in the proportions as for outer shell. The mix was melted and tempered. Consequently, Phytrol/soybean oil blend was mixed with chocolate in the 1:1 ratio and deposited into molds previously filled with chocolate without Phytrol. Chocolate pieces were than cooled, wrapped and packed into the boxes. Using the molding system, 10-12 g chocolate pieces were produced.

### Example: 10 Softgel Capsule Dosage Form.

Phytosterols were dissloved in an edible oil carrier using the protocol outlined in Example 1 and subsequently mixed with a dispersing/emulsifying agent and lecithin, in combination with a medium chain monoglyceride (di or triglycerides, or combinations thereof, may also be used). Depending on the purpose for which the combination product is used, the necessary dosage was supplied in one or two capsules taken with each meal.

### Example: 11 Oral Microemulsion.

Phytosterols were dissolved in an edible oil using the protcol of Example 1 and then the composition was mixed with appropriate excipients to form a self-emulsifying drug delivery system which presented itself as a microemulsion in the gastrointestinal fluids. Suitable excipients comprised a blend of medium chain mono- and diglycerides having HLB values within the range 2-7, e.g. the CAPMUL (trademark) series; a medium chain triglyceride, e.g. a member of the CAPTEX (trademark) series; a high HLB emulsifier (HLB value 10-16), e.g. polysorbate 20 ; and water. Appropriate flavouring agents, preservatives and anti-oxidants were also incorporated. Depending on the purpose for which the combination product is used, the necessary dosage would be supplied in 5-10 mL of preparation taken with each meal.

### REFERENCES

1. Law M.R., Wald N.J., Wu., Hacksaw ZA., Bailey A.; Systemic underestimation of association between serum cholesterol concentration and ischemic heart disease in observational studies: Data from BUPA Study; *Br. Med. J*. 1994; 308:363-366
2. Law M.R., Wald N.J., Thompson S.G.; By how much and how quickly does reduction in serum cholesterol concentration lower risk of ischemic heart disease? *Br. Med. J*. 1994; 308:367-373
3. La Rosa J.C., Hunninghake D.. Bush D. et al.; The cholesterol facts: A summary of the evidence relating to dietary fats, serum cholesterol and coronary heart disease:Ajoint statement by the American Heart Association and the National Heart, Lung and Blood Institute. *Circulation* 1990; 81:1721-1733
4. Havel R.J., Rapaport E.. Drug Therapy: Management of Primary Hyperlipidemia. *New England Journal of Medicine,* 1995; 332:1491-1498
5. Kuccodkar et al.; Effects of plant sterols on cholesterol metabolism. *Atherosclerosis,* 1976; 23:239-248
6. Lees R.S., Lees A.M. Effects of sitosterol therapy on plasma lipid and lipoprotein concentrations. In: Greten H (Ed) Lipoprotein Metabolism. Springer-Verlag, Berlin, Heidelberg, New York, 1976:119-124
7. Lees A.M., Mok H.Y.I., Lees R.S., McCluskey M.A., Grundy S.M. Plant sterols as cholesterol-lowering agents: clinical trials in patients with hypercholesterolemia and studies of sterol balance. *Atherosclerosis* 1977; 28: 325-338
7a. Mattson FH et al.: American Journal of Clinical Nutrition. 35(4):697-700, 1982

## Claims

1. A method of preparing a composition comprising an edible oil or fat and one of phytosterols and/or phytostanols and in which the phytosterols and/or phytostanols are substantially completely dissolved therein comprises the steps of:
a) heating the phytosterols and/or phytostanols at least to the melting temperature of the phytosterols and/or phytostanols and with no other material present to form a molten material;
b) heating the edible oil or fat;
c) mixing the molten material with the heated edible oil or fat; and
d) cooling the composition so formed.

2. The method of claim 1 wherein the oil is any edible oil of plant or animal origin and the fat is any animal fat.

3. The method of claim 1 wherein the oil is selected from the group consisting of sunflower oil, rapeseed oil, soybean oil, olive oil, corn oil, safflower oil, sesame seed oil.

4. The method of claim 1 wherein the phytosterols are selected from the group consisting of sitosterol, campesterol, stigmasterol, brassicasterol, desmosterol, chalinosterol, poriferasterol, clionasterol and all natural or synthesized forms and derivatives thereof, including isomers.

5. The method of claim 1 wherein the phytostanols are selected from the group consisting of sitostanol, campestanol, brassicastanol, desmostanol, chalinostanol, poriferastanol, clionastanol and all natural or synthesized forms and derivatives thereof, including isomers.

6. The method of claim 1 wherein the phytosterols and/or phytostanols are heated at step a) to a temperature of between 120-145°C.

7. The method of claim 1 wherein the edible oil or fat is heated at step b) to a temperature of between 80-150°C.

8. A composition comprising an edible oil or fat and one or more phytosterols and/or phytostanols, wherein the phytosterols and/or phytostanols are substantially completely dissolved therein by a method in which the phytosterols and/or phytostanols are heated at least to the melting temperature of the phytosterols and/or phytostanols and with no other material present to form a molten material which is then added to a heated oil or fat and the composition so formed is cooled.

9. The composition of claim 8 wherein the oil is any edible oil of plant or animal origin and the fat is any animal fat.

10. The composition of claim 8 wherein the oil is selected from the group consisting of sunflower oil, rapeseed oil, soybean oil, olive oil, corn oil, safflower oil, sesame seed oil.

11. The composition of claim 8 wherein the phytosterols are selected from the group consisting of sitosterol, campesterol, stigmasterol, brassicasterol, desmosterol, chalinosterol, poriferasterol, clionasterol and all natural or synthesized forms and derivatives thereof, including isomers.

12. The composition of claim 8 wherein the phytostanols are selected from the group consisting of sitostanol, campestanol, brassicastanol, desmostanol, chalinostanol, poriferastanol, clionastanol and all natural or synthesized forms and derivatives thereof, including isomers.

13. The composition of claim 8 wherein the phytosterols and/or phytostanols are heated to a temperature of between 120-145°C to produce the molten material.

14. The composition of claim 8 wherein the edible oil or fat is heated to a temperature of between 80-150°C prior to addition thereto of the molten material.

15. The composition of any of claims 8 to 14 comprising 1 to 30% by weight phytosterol and 99 to 70% oil or fat.

16. The composition according to claim 14 or claim 15 comprising at least 5% by weight phytosterol.

17. A food product comprising the composition of claim 8.

18. A beverage comprising the composition of claim 8.

19. A pharmaceutical product comprising the composition of claim 8.

20. Use of the composition of any of claims 8 to 16 in the manufacture of a product for administration to an animal for the treatment or prevention of cardiovascular disease or its underlying conditions.

## Patentansprüche

1. Verfahren zur Herstellung einer Zusammensetzung, umfassend ein Speiseöl oder -fett und eines von Phytosterolen und/oder Phytostanolen, in der die Phytosterole und/oder Phytostanole im wesentlichen vollständig darin gelöst sind, umfassend die Schritte:
a) Erwärmen der Phytosterole und/oder Phytostanole zumindest auf die Schmelztemperatur der Phytosterole und/oder Phytostanole, ohne dass irgendein anderes Material zugegen ist, um ein geschmolzenes Material zu bilden;
b) Erwärmen des Speiseöls oder -fetts,
c) Mischen des geschmolzenen Materials mit dem erwärmten Speiseöl oder -fett; und
d) Kühlen der so gebildeten Zusammensetzung.

2. Verfahren nach Anspruch 1, worin das Öl irgendein Speiseöl pflanzlichen oder tierischen Ursprungs ist und das Fett irgendein Tierfett ist.

3. Verfahren nach Anspruch 1, worin das Öl ausgewählt ist aus der Gruppe bestehend aus Sonnenblumenöl, Rapsöl, Sojaöl, Olivenöl, Maisöl, Safloröl, Sesamöl.

4. Verfahren nach Anspruch 1, worin die Phytosterole ausgewählt sind aus der Gruppe bestehend aus Sitosterol, Campesterol, Stigmasterol, Brassicasterol, Desmosterol, Chalinosterol, Poriferasterol, Clionasterol und allen natürlichen oder künstlichen Formen und Derivaten davon, einschließlich Isomere.

5. Verfahren nach Anspruch 1, worin die Phytostanole ausgewählt sind aus der Gruppe bestehend aus Sitostanol, Campestanol, Brassicastanol, Desmostanol, Chalinostanol, Poriferastanol, Clionastanol und allen natürlichen oder künstlichen Formen und Derivaten davon, einschließlich Isomere.

6. Verfahren nach Anspruch 1, worin die Phytosterole und/oder Phytostanole in Schritt a) auf eine Temperatur zwischen 120 und 145°C erwärmt werden.

7. Verfahren nach Anspruch 1, worin das Speiseöl oder -fett in Schritt b) auf eine Temperatur zwischen 80 und 150°C erwärmt wird.

8. Zusammensetzung, umfassend ein Speiseöl oder -fett und ein oder mehrere Phytosterole und/oder Phytostanole, wobei die Phytosterole und/oder Phytostanole durch ein Verfahren, bei dem die Phytosterole und/oder Phytostanole zumindest auf die Schmelztemperatur der Phytosterole und/oder Phytostanole erwärmt werden, ohne dass irgendein anderes Material zugegen ist, um ein geschmolzenes Material zu bilden, welches dann zu einem erwärmten Öl oder Fett gegeben wird, und die so gebildete Zusammensetzung gekühlt wird, im wesentlichen vollständig darin gelöst sind.

9. Zusammensetzung nach Anspruch 8, worin das Öl irgendein Speiseöl pflanzlichen oder tierischen Ursprungs ist und das Fett irgendein Tierfett ist.

10. Zusammensetzung nach Anspruch 8, worin das Öl ausgewählt ist aus der Gruppe bestehend aus Sonnenblumenöl, Rapsöl, Sojaöl, Olivenöl, Maisöl, Safloröl, Sesamöl.

11. Zusammensetzung nach Anspruch 8, worin die Phytosterole ausgewählt sind aus der Gruppe bestehend aus Sitosterol, Campesterol, Stigmasterol, Brassicasterol, Desmosterol, Chalinosterol, Poriferasterol, Clionasterol und allen natürlichen oder künstlichen Formen und Derivaten davon, einschließlich Isomere.

12. Zusammensetzung nach Anspruch 8, worin die Phytostanole ausgewählt sind aus der Gruppe bestehend aus Sitostanol, Campestanol, Brassicastanol, Desmostanol, Chalinostanol, Poriferastanol, Clionastanol und allen natürlichen oder künstlichen Formen und Derivaten davon, einschließlich Isomere.

13. Zusammensetzung nach Anspruch 8, wobei die Phytosterole und/oder Phytostanole auf eine Temperatur zwischen 120 und 145°C erwärmt werden, um das geschmolzene Material zu bilden.

14. Zusammensetzung nach Anspruch 8, wobei das Speiseöl oder -fett auf eine Temperatur zwischen 80 und 150°C erwärmt wird, bevor das geschmolzene Material dazu gegeben wird.

15. Zusammensetzung nach irgendeinem der Ansprüche 8 bis 14, umfassend 1 bis 30 Gew.-% Phytosterol und 99 bis 70% Öl oder Fett.

16. Zusammensetzung nach Anspruch 14 oder Anspruch 15, umfassend mindestens 5 Gew.-% Phytosterol.

17. Lebensmittelprodukt, umfassend die Zusammensetzung nach Anspruch 8.

18. Getränk, umfassend die Zusammensetzung nach Anspruch 8.

19. Pharmazeutisches Produkt, umfassend die Zusammensetzung nach Anspruch 8.

20. Verwendung der Zusammensetzung nach irgendeinem der Ansprüche 8 bis 16 bei der Herstellung eines Produkts zur Verabreichung an ein Tier zur Behandlung oder Vermeidung von kardiovaskulärer Erkrankung oder deren zugrundeliegenden Zuständen.

## Revendications

1. Procédé de préparation d'une composition comprenant une huile ou une matière grasse comestible et un des phytostérols et/ou des phytostanols, et dans laquelle les phytostérols et/ou les phytostanols sont sensiblement totalement dissous dans la composition, qui comprend les étapes consistant à :
a) chauffer les phytostérols et/ou les phytostanols à au moins la température de fusion des phytostérols et/ou des phytostanols et en l'absence d'autre matériau présent, pour former un matériau fondu ;
b) chauffer l'huile ou la matière grasse comestible ;
c) mélanger le matériau fondu avec l'huile ou la matière grasse comestible chauffée ; et
d) refroidir la composition ainsi formée.

2. Procédé selon la revendication 1, dans lequel l'huile est n'importe quelle huile comestible d'origine animale ou végétale et la matière grasse est n'importe quelle matière grasse animale.

3. Procédé selon la revendication 1, dans lequel l'huile est choisie dans le groupe comprenant l'huile de tournesol, l'huile de colza, l'huile de soja, l'huile d'olive, l'huile de maïs, l'huile de carthame et l'huile de sésame.

4. Procédé selon la revendication 1, dans lequel les phytostérols sont choisis dans le groupe comprenant le sitostérol, le campestérol, le stigmastérol, le brassicastérol, le desmostérol, le chalinostérol, le poriférastérol, le clionastérol et toutes les formes naturelles ou synthétisées et des dérivés de ceux-ci, y compris les isomères.

5. Procédé selon la revendication 1, dans lequel les phytostanols sont choisis dans le groupe comprenant le sitostanol, le campestanol, le brassicastanol, le desmostanol, le chalinostanol, le poriférastanol, le clionastanol et toutes les formes naturelles ou synthétisées et des dérivés de ceux-ci, y compris les isomères.

6. Procédé selon la revendication 1, dans lequel les phytostérols et/ou les phytostanols sont chauffés à l'étape a) à une température comprise dans la plage allant de 120 à 145 °C.

7. Procédé selon la revendication 1, dans lequel l'huile ou la matière grasse comestible est chauffée à l'étape b) à une température comprise dans la plage allant de 80 à 150 °C.

8. Composition comprenant une huile ou une matière grasse comestible et un ou plusieurs phytostérols et/ou phytostanols, dans laquelle les phytostérols et/ou les phytostanols sont sensiblement totalement dissous dans la composition par un procédé dans lequel les phytostérols et/ou les phytostanols sont chauffés à au moins la température de fusion des phytostérols et/ou des phytostanols et en l'absence d'autre matériau présent, pour former un matériau fondu qui est ensuite ajouté à une huile ou une matière grasse chauffée et la composition formée est refroidie.

9. Composition selon la revendication 8, dans laquelle l'huile est n'importe quelle huile comestible d'origine animale ou végétale et la matière grasse est n'importe quelle matière grasse animale.

10. Composition selon la revendication 8, dans laquelle l'huile est choisie dans le groupe comprenant l'huile de tournesol, l'huile de colza, l'huile de soja, l'huile d'olive, l'huile de maïs, l'huile de carthame et l'huile de sésame.

11. Composition selon la revendication 8, dans laquelle les phytostérols sont choisis dans le groupe comprenant le sitostérol, le campestérol, le stigmastérol, le brassicastérol, le desmostérol, le chalinostérol, le poriférastérol, le clionastérol et toutes les formes naturelles ou synthétisées et des dérivés de ceux-ci, y compris les isomères.

12. Composition selon la revendication 8, dans laquelle les phytostanols sont choisis dans le groupe comprenant le sitostanol, le campestanol, le brassicastanol, le desmostanol, le chalinostanol, le poriférastanol, le clionastanol et toutes les formes naturelles ou synthétisées et des dérivés de ceux-ci, y compris les isomères.

13. Composition selon la revendication 8, dans laquelle les phytostérols et/ou les phytostanols sont chauffés à une température comprise dans la plage allant de 120 à 145 °C pour produire le matériau fondu.

14. Composition selon la revendication 8, dans laquelle l'huile ou la matière grasse comestible est chauffée à une température comprise dans la plage allant de 80 à 150 °C avant l'ajout du matériau fondu.

15. Composition selon l'une quelconque des revendications 8 à 14, comprenant de 1 à 30 % en poids de phytostérol et de 99 à 70 % d'huile ou de matière grasse.

16. Composition selon la revendication 14 ou 15, comprenant au moins 5 % en poids de phytostérol.

17. Produit alimentaire comprenant la composition selon la revendication 8.

18. Boisson comprenant la composition selon la revendication 8.

19. Produit pharmaceutique comprenant la composition selon la revendication 8.

20. Utilisation de la composition selon l'une quelconque des revendications 8 à 16 pour la fabrication d'un produit à administrer à un animal, destiné au traitement ou à la prévention d'une maladie cardiovasculaire ou de ses pathologies sous-jacentes.
